# FASCICULE DE BREVET EUROPEEN

(11) **EP 4 449 113 B1**
(45) Date de publication et mention de la délivrance du brevet: **01.07.2026**
(21) Numéro de dépôt: 22847557.0
(22) Date de dépôt: 15.12.2022
(51) Int. Cl.: G01N 31/22, C07D 215/12, G01N 21/78, G03F 7/033

(54) **UTILISATION D'UN COMPOSE SPECIFIQUE POUR LA DETECTION DE COMPOSES CHIMIQUES TOXIQUES**
VERWENDUNG EINER SPEZIFISCHEN VERBINDUNG ZUM NACHWEIS TOXISCHER CHEMISCHER VERBINDUNGEN
USE OF A SPECIFIC COMPOUND FOR THE DETECTION OF TOXIC CHEMICALS

(30) Priorité: 16.12.2021 FR 2113676
(43) Date de publication de la demande: 23.10.2024
(73) Titulaire: Commissariat à l'Energie Atomique et aux Energies Alternatives, 75015 Paris (FR)
(72) Inventeur: TERMEAU, Lucile, 38054 Grenoble Cedex 09 (FR); PENLOU, Sébastien, 38054 Grenoble Cedex 09 (FR); CARELLA, Alexandre, 38054 Grenoble Cedex 09 (FR)
(74) Mandataire: Santarelli
(86) Numéro de dépôt international: PCT/FR2022/052381
(87) Numéro de publication internationale: WO 2023/111471

(56) Documents cités:
- CN-B- 110 015 992
- US-A- 5 616 502
- GERHARD J MOHR: "New chromogenic and fluorogenic reagents and sensors for neutral and ionic analytes based on covalent bond formation-a review of recent developments", ANALYTICAL AND BIOANALYTICAL CHEMISTRY, SPRINGER, BERLIN, DE, vol. 386, no. 5, 13 October 2006 (2006-10-13), pages 1201 - 1214, XP019441119, ISSN: 1618-2650, DOI: 10.1007/S00216-006-0647-3
- PENG JUANJUAN ET AL: "Gold nanoparticle-based detection of dopamine based on fluorescence resonance energy transfer between a 4-(4-dialkylaminostyryl)pyridinium derived fluorophore and citrate-capped gold nanoparticles", MICROCHIMICA ACTA, SPRINGER VIENNA, VIENNA, vol. 186, no. 9, 13 August 2019 (2019-08-13), XP036904346, ISSN: 0026-3672, [retrieved on 20190813], DOI: 10.1007/S00604-019-3727-8
- DE C ET AL: "Dual colorimetric and electrochemical sensing of organothiophosphorus pesticides by an azastilbene derivative", TETRAHEDRON LETTERS, ELSEVIER, AMSTERDAM , NL, vol. 51, no. 13, 31 March 2010 (2010-03-31), pages 1754 - 1757, XP026920722, ISSN: 0040-4039, [retrieved on 20100201], DOI: 10.1016/J.TETLET.2010.01.100

## Description

### DOMAINE TECHNIQUE

La présente invention a trait à l'utilisation de nouveaux composés spécifiques pour la détection de composés organiques appartenant à la catégorie des composés chimiques toxiques et, plus spécifiquement, à la catégorie des composés organophosphorés, des composés sulfures, des composés amines et des composés arséniés, ces composés pouvant être des composés toxiques de guerre, des pesticides, des vésicants, des composés toxiques industriels chimiques (connus sous la dénomination TIC).

De manière générale, les composés organophosphorés se présentent sous la forme de composés organiques présentant une toxicité avérée pour l'organisme humain. En effet, ces composés peuvent être impliqués dans le mécanisme d'inhibition des sérineprotéases et, en particulier, de l'acétylcholinestérase, qui intervient dans les jonctions synaptiques et dont le dérèglement de l'activité peut empêcher le relâchement musculaire et ainsi provoquer la mort par asphyxie.

Ces composés peuvent être compris dans la formulation d'insecticides, de pesticides ou encore d'agents chimiques de combat (tels que les composés organosphosphorés de la série G, comme le sarin ou les composés organophosphorés de la série V, comme le VX) et du fait de la létalité élevée de ces composés, de leur prolifération, il s'avère important de pouvoir disposer de systèmes de détection.

Certains systèmes de détection utilisés à ce jour sont basés sur des technologies impliquant des moyens de mesure physique, telles que la spectroscopie à mobilité ionique, la photométrie de flamme, les spectroscopies IR et Raman, avec, pour difficultés, que ces systèmes nécessitent un appareillage complexe onéreux et pas forcément adaptés à tous les milieux d'intervention en termes de masse et d'encombrement, auxquelles s'ajoute l'expertise de l'opérateur à considérer.

Au vu de ce qui existe, les auteurs de la présente invention se sont orientés vers la découverte de nouveaux composés utilisables pour la détection de composés chimiques, tels que des composés organophosphorés, avec une application tant en milieu liquide qu'en milieu supporté et permettant une détection simple.

Le document US 5 616 502 décrit l'utilisation d'une variété de colorants mérocyanines et de colorants mérocyanines substitués pour détecter et quantifier les poly(acides aminés), y compris les peptides, les polypeptides et les protéines. L'article GERHARD J MOHR « New chromogenic and fluorogenic reagents and sensors for neutral and ionic analytes based on covalent bond formation - a review of recent developements » Analytical and Bioanalytical Chemistry, vol. 386, n°5, p.1201-1214 décrit des nouveaux composés pour la détection d'amines, de diamines, d'acides aminés et de protéines.

### EXPOSÉ DE L'INVENTION

La presente invention est définie dans les revendications annexées. L'invention a trait à l'utilisation d'un composé comprenant un groupe aromatique comprenant au moins un cycle aromatique azoté, lequel cycle étant porteur d'au moins deux substituants qui répondent à la formule (I) suivante : 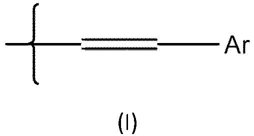 dans laquelle Ar désigne un groupe aromatique éventuellement substitué et l'accolade désigne l'endroit par lequel le substituant est lié au cycle aromatique azoté,
ledit composé étant utilisé comme indicateur de détection pour la détection d'au moins un composé chimique choisi parmi les composés organophosphorés, les composés sulfures, les composés amines et les composés arséniés.

Par indicateur de détection, il s'entend, classiquement, un composé chimique capable de se transformer au contact d'au moins un autre composé chimique que l'on souhaite détecter, cette transformation étant le signe de la présence dudit au moins un autre composé chimique, cette transformation se matérialisant par un changement de couleur, lorsque l'indicateur de détection est un indicateur coloré, ce qui est le cas de tout ou partie des composés objets de l'utilisation de l'invention. Plus spécifiquement, lorsque l'indicateur de détection est un indicateur coloré, celui-ci correspond, classiquement, à un composé chimique qui prend au moins une couleur caractéristique en présence d'un autre composé chimique soit, en d'autres termes, un composé chimique qui présente au moins deux états colorés, un état coloré existant lorsque la substance chimique n'est pas en présence du composé chimique à détecter et au moins un autre état coloré lorsque le composé chimique est en présence du composé chimique à détecter.

Comme mentionné ci-dessus, le composé utilisé, comme indicateur de détection, est un composé comprenant au moins un groupe aromatique comprenant au moins un cycle aromatique azoté, lequel cycle étant porteur d'au moins deux substituants dont l'un au moins des substituants comprend une double liaison conjuguée avec ledit cycle.

Plus spécifiquement, le composé peut être un composé comprenant, comme groupe aromatique comprenant au moins un cycle aromatique azoté, un groupe aromatique comprenant un seul cycle aromatique azoté.

Encore plus spécifiquement, le composé peut être un composé comprenant, comme groupe aromatique comprenant au moins un cycle aromatique azoté, un groupe aromatique comprenant, comme cycle aromatique azoté, un cycle pyridine, lequel groupe aromatique pouvant être un groupe pyridine en tant que tel (ce qui signifie, en d'autres termes ; que le groupe aromatique est constitué d'un seul cycle aromatique azoté, lequel est un cycle pyridine) ou un groupe quinoléine.

Le cycle aromatique azoté peut être porteur de deux substituants uniquement, dont l'un au moins des substituants comprend une double liaison conjuguée avec ledit cycle et, de préférence, deux substituants identiques, autrement deux substituants identiques comprenant chacun une double liaison conjuguée avec ledit cycle.

Sans être lié par la théorie, le cycle aromatique azoté est impliqué, en grande partie, dans la détection des composés mentionnés ci-dessus, avec une préférence pour une disubstitution par des parties conjuguées qui semble être responsable de la grande différence de couleur entre l'état initial du composé et son état final après exposition au contact du composé à détecter, cette conjugaison pouvant être engendrée par la présence d'une double liaison en position alpha du cycle aromatique azoté.

Ar désignant un groupe aromatique éventuellement substitué et l'accolade désignant l'endroit par lequel le substituant est lié au cycle aromatique azoté, cette formule couvrant tant les formes isomères *trans* que *cis.*

Le groupe aromatique de tout ou partie des substituants et, en particulier Ar de la formule (I) ci-dessus mentionnée, peut être un groupe aromatique monocyclique carboné, par exemple, un groupe phényle ou un groupe hétéroaromatique monocyclique, par exemple, un groupe hétéroaromatique azoté monocyclique, tel qu'un groupe pyridine.

Le groupe aromatique de tout ou partie des substituants et, en particulier Ar de la formule (I) ci-dessus mentionnée, peut être éventuellement substitué par un ou plusieurs substituants, par exemple, choisi parmi un substituant amine (par exemple, une amine primaire -NH₂, une amine tertiaire alkylée, comme -N(CH₃)₂), un substituant alcoxy (par exemple, -OCH₃), un substituant hydroxyle -OH, un substituant nitro -NO₂, un substituant halogène, un substituant alkyle halogéné (par exemple, un substituant perhalogénoalkyle, comme -CF₃), un substituant ester, tel que -COOCH₃, un substituant acide carboxylique -COOH, un substituant cyano -CN ou un substituant -S-alkyle (par exemple, -SCH₃).

Selon un mode particulier de l'invention, le composé peut être un composé comprenant, comme groupe aromatique comprenant au moins un cycle aromatique azoté, un groupe quinoléine, le cycle aromatique azoté du groupe quinoléine étant porteur de deux substituants identiques comprenant chacun une double liaison conjuguée avec ledit cycle et comprenant chacun un groupe aromatique conjugué avec ladite double liaison. Un composé de ce type peut répondre à la formule (II) suivante : 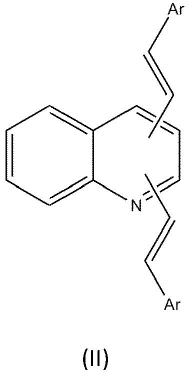 dans laquelle Ar désigne un groupe aromatique, plus spécifiquement, un groupe aromatique monocyclique, par exemple, un groupe phényle ou un groupe pyridine, les deux substituants pouvant être liés à n'importe quel des atomes de carbone libre du cycle pyridine ( à savoir, dans ce cas, les positions *ortho, meta* ou *para*)*,* ledit groupe aromatique étant éventuellement substitué par un ou plusieurs substituants, par exemple, choisi parmi un substituant amine (par exemple, une amine primaire -NH₂, une amine tertiaire alkylée, comme -N(CH₃)₂), un substituant alcoxy (par exemple, -OCH₃), un substituant hydroxyle -OH, un substituant nitro -NO₂, un substituant halogène, un substituant alkyle halogéné (par un substituant perhalogénoalkyle, comme -CF₃), un substituant ester, tel que -COOCH₃, un substituant acide carboxylique -COOH, un substituant cyano -CN ou un substituant -S-alkyle (par exemple, -SCH₃).

Plus spécifiquement, un composé de ce type peut être un composé, dans lequel l'un des substituants occupe la position *ortho* et l'autre substituant occupe la position *para* (par rapport à l'azote), ce composé répondant ainsi à la formule (III) suivante : 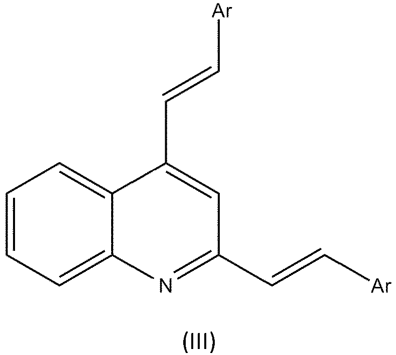 avec Ar étant tel que défini ci-dessus.

En particulier, Ar peut être un groupe phényle substitué par un groupe amine et, plus spécifiquement un groupe amine tertiaire, tel que -N(CH₃)₂ situé, en particulier, en position *para,* un tel composé répondant à la formule (IV) suivante : 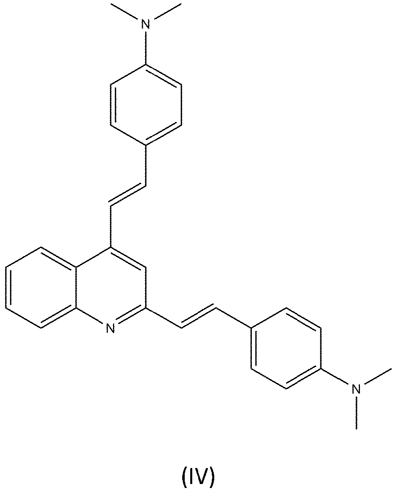 ce composé correspondant à la 2,4-*bis*[p-(diméthylamino)styryl)quinoléine.

Les composés utilisés dans le cadre de l'invention peuvent être achetés chez un fournisseur ou peuvent être synthétisés par des techniques classiques de synthèse organique impliquant, par exemple, des réactions de couplage, comme un couplage de Heck, un couplage de Suzuki, des réactions impliquant l'utilisation de micro-ondes.

Par exemple, lorsque le composé répond à la formule (IV) mentionnée ci-dessus, une voie de synthèse peut consister en la formation d'un composé intermédiaire sous forme d'un acide boronique suivie d'un couplage de Suzuki comme illustré sur le schéma suivant :

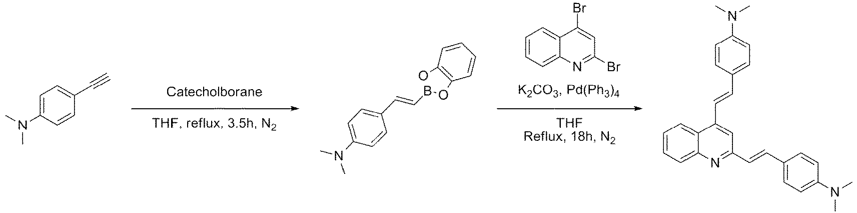

Comme mentionné ci-dessus, les composés utilisés dans le cadre de l'invention font office d'indicateur de détection, en particulier colorimétrique, pour la détection d'un composé choisi parmi les composés organophosphorés, les composés sulfures, les composés amines et les composés arséniés.

Plus spécifiquement, les composés aptes à être détectés, notamment avec le composé de formule (IV) ci-dessus mentionnée, peuvent être :
- comme composés organophosphorés, des pesticides, tels que le malathion, le phosalone, le trichlorfon, l'ométhoate, le fenthion, le parathion, le chlorpyrifos et le paraoxon méthyl ou des composés toxiques de guerre, tels que le sarin, le tabun, le phosphonamidofluoridate de méthyl(1-(diéthylamino)éthylidène), le phosphoramidofluoridate d'éthyl(1-(diéthylamino)éthylidène), le (bis(diéthylamino)méthylène)phosphoramidofluoridate de méthyle ;
- comme composés sulfures, un composé toxique de guerre, tel que l'ypérite soufrée ;
- comme composés amines, un composé toxique de guerre, tel que l'ypérite azotée ;
- comme composés arséniés, des composés toxiques de guerre, tels que la léwisite, la diphénylchlorarsine, la diphénylcyanoarsine.

Les composés utilisés dans le cadre de l'invention pour la détection peuvent être utilisés en phase liquide ou en phase supportée.

Lorsqu'ils sont utilisés en phase supportée, le support peut être un support en papier (tel que du papier chromatographie), un support en fibres intissées (par exemple, un mélange de fibres de cellulose et de fibres de polyester), étant entendu que le support doit être apte à être imprégné par le composé retenu pour la détection soit directement soit *via* des particules préalablement imprégnées par le composé retenu pour la détection.

Les supports susmentionnés peuvent être préparés par dépôt du composé retenu d'une solution comprenant ledit composé soit à l'aide d'une micropipette, soit par impression (par exemple, *via* une imprimante Dimatix) ou par dépôt par sérigraphie.

Les supports susmentionnés peuvent être préparés également par dépôt sur un support d'une composition comprenant des particules imprégnées préalablement par le composé indicateur de détection suivi d'un séchage de ladite composition.

Les types distincts de particules peuvent être des particules inorganiques, des particules organiques ou des particules hybrides organiques-inorganiques, par exemple, des particules dites « cœur-écorce » avec un cœur inorganique et une écorce organique.

A titre d'exemples de particules inorganiques, il peut être fait mention :
- de particules de silice, en particulier les particules de silice fournies par Aldrich sous la référence de produit 236802, ces particules de silice présentant une taille moyenne de pores de 60 Å ;
- de particules d'alumine ;
- de particules d'oxyde de zinc ;
- de particules d'oxyde de titane ;
- de particules de terre de diatomée ;
- de particules de zéolithe ;
- de particules en géopolymère.

A titre d'exemples de particules organiques, il peut être fait mention de particules polymériques, telles que :
- de particules de polyéthylène, en particulier, les particules de polyéthylène fournies par Aldrich sous la référence de produit 434264, ces particules de polyéthylène présentant une taille moyenne de particules de 180 µm ou les particules de polyéthylène fournies par Alfa sous la référence de produit A10239 présentant une taille moyenne de particules de 500 µm ou encore les particules de polyéthylène fournies par Mitsui Chemicals sous la référence de produit MIPELON XM220 présentant une taille moyenne de particules de 30 µm ;
- de particules de polyamide, telles que des particules de nylon, en particulier, les particules de nylon-6 fournies par Aldrich sous la référence de produit GF58818471 présentant une taille moyenne de particules allant de 5 à 50 µm ou les particules de nylon-12 fournies par Aldrich sous la référence de produit GF33201098 présentant une taille moyenne de particules allant de 10 à 50 µm ;
- de particules d'amidon.

Enfin, l'invention a trait également à un procédé de détection de la présence ou de l'absence d'au moins un composé chimique choisi parmi les composés organophosphorés, les composés sulfures, les composés amines et les composés arséniés comprenant les étapes suivantes :
- une étape de mise en contact du ou des milieux, dont on veut détecter la présence ou l'absence du ou desdits composés chimiques avec un indicateur de détection qui est un composé tel que défini ci-dessus ;
- une étape de déduction, en fonction du ou des éventuels signaux de transformation de l'indicateur de détection, de la présence ou de l'absence du ou desdits composés, ce ou ces signaux de transformation étant classiquement un virage chromatique.

Cette étape de mise en contact peut consister à déposer en surface du système de détection une ou plusieurs gouttes distinctes de chaque milieu dont on veut analyser l'absence ou la présence d'un ou plusieurs composés chimiques.

Il s'entend que le composé devant être utilisé dans le cadre du procédé susmentionné doit être apte à détecter le ou les composés chimiques, dont on veut déterminer l'absence ou la présence.

Entre l'étape de mise en contact et l'étape de déduction, il peut être prévu un temps d'attente pour que, le cas échéant, le virage chromatique puisse avoir lieu.

Concernant l'étape de déduction, lorsque le ou les signaux de transformation consiste(nt) classiquement en un virage chromatique, l'opérateur pourra se baser sur une échelle colorimétrique associée au composé faisant office d'indicateur de détection, qui définira, pour tous les composés chimiques susceptibles d'être détectés par le système, le virage chromatique correspondant, cette échelle colorimétrique pouvant être déterminée, par des essais préalables, pour l'indicateur de détection et les composés chimiques destinés à être détectés par ledit indicateur.

D'autres caractéristiques et avantages de l'invention apparaîtront mieux à la lecture du complément de description qui suit, lequel à des exemples de détection conformes à l'invention.

Bien entendu, les exemples qui suivent ne sont donnés qu'à titre d'illustration de l'objet de l'invention et ne constituent en aucun cas une limitation de cet objet.

### EXPOSÉ DÉTAILLÉ DE MODES DE RÉALISATION PARTICULIERS

### EXEMPLE 1

Cet exemple illustre la préparation d'un composé spécifique utilisable dans le cadre de l'invention, la 2-4*-bis*[p-(diméthylamino)styryl]quinoléine répondant à la formule suivante : 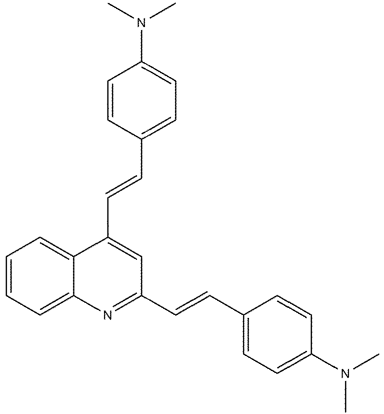 ce composé étant préparé par les étapes résumées sur le schéma suivant :

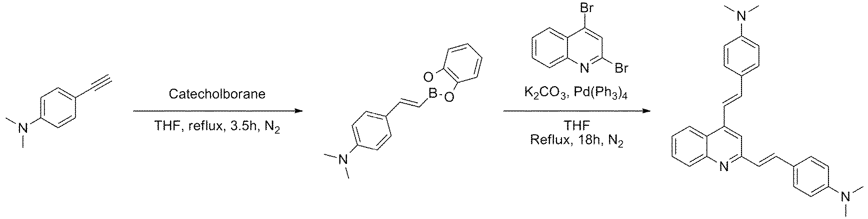

Le protocole détaillé est le suivant.

La 4-éthynyl-N,N-diméthylaniline (290,4 mg, 2 mmol) est solubilisée dans 20 mL de THF. La solution est dégazée avec de l'argon et mis sous atmosphère inerte. Une solution de catécholborane à 1M dans du THF (2,4 mL, 2,4 mmol) est ajoutée au mélange et la réaction est portée à reflux (60°C) pendant 1,5 heures. Une 2^{ème} portion de catécholborane (1,6 mL, 1,6 mmol) est ajoutée et la réaction est agitée à reflux pendant 2 heures supplémentaires. Le mélange refroidi à température ambiante (25°C) et la 2,4-dibromoquinoléine (229,6 mg, 2 mmol) et le catalyseur Pd(PPh₃)₄ (462,2 mg, 0,4 mmol) sont directement ajoutés dans le milieu réactionnel sous agitation magnétique. Après 20 minutes, une solution aqueuse à 20% de K₂CO₃ (1 mL) est ajoutée et la réaction est portée à reflux pour un temps de réaction total de 18 heures toujours sous atmosphère inerte (Ar). Après ce temps, le mélange est refroidi à température ambiante et 5 mL d'eau déionisée sont ajoutés. Le composé est extrait par de l'acétate d'éthyle (2 x 10 mL). Les phases organiques sont combinées, lavées avec de l'eau (2 x 10 mL) et de la saumure de chlorure de sodium (10 mL) et séchées sur MgSO₄. L'excès de solvant est retiré par évaporation et le composé est purifié par chromatographie sur colonne de silice.

### EXEMPLE 2

Cet exemple illustre la mise en œuvre du composé préparé à l'exemple 1 pour la détection en phase liquide de composés pesticides organophosphorés ou organothiophosphorés.

Pour ce faire, la 2,4-*bis-*[p-(dimethylamino)styryl]quinoléine préparé à l'exemple 1 est solubilisée à 10 mM dans du DMSO. La molécule prend ainsi une couleur jaune à l'état initial. Les pesticides sont quant à eux solubilisés à 0,267 M. La phase d'exposition consiste à prélever 2,5 µL de la solution de 2,4-bis[p-(dimethylamino)styryl]quinoléine, de diluer ce volume par 10 µL de DMSO et d'y ajouter 37,5 µL de la solution d'un pesticide donné. Ainsi, la 2,4-*bis*[p-(dimethylamino)styryl]quinoléine se retrouve dans un volume final de 50 µL de DMSO, concentré à 0,5 mM en présence de 400 équivalents de pesticide (à la concentration finale de 0,01 mM). La 2,4-*bis*[p-(dimethylamino)styryl]quinoléine a ainsi été exposée à 8 pesticides : Malathion, Phosalone, Trichlorfon, Ométhoate, Fenthion, Parathion, Chlorpyrifos et paraoxon méthyl.

Il ressort que le 2,4-*bis*[p-(dimethylamino)styryl]quinoléine subit un virage colorimétrique visible à l'œil nu pour l'ensemble des pesticides testés avec une cinétique en fonction des pesticides, la cinétique étant notamment plus rapide avec le malathion et le trichlorfon.

### EXEMPLE 3

Cet exemple illustre la mise en œuvre du composé préparé à l'exemple 1 pour la détection en phase liquide de composés toxiques de guerre.

Pour ce faire, 100 µL de 2,4-*bis*[p-diméthylamino)styryl]-quinoléine en solution à 1 mmol/L dans le DMSO est déposé à l'aide d'une pipette électronique multicanaux dans des puits d'une plaque en polystyrène.

Cinq toxiques de guerre (Sarin (dit GB, CAS n°107-44-8), Tabun (dit GA, CAS n°77-81-6), Ypérite soufrée (dite HD, CAS n°505-60-2), Ypérite azotée (dite HN3, CAS n°555-71-1), Léwisite (dite L1, CAS n°541-25-3)) sont déposés dans des puits distincts sous forme de gouttes à l'aide d'une pipette électronique multicanaux. Les volumes de toxiques déposés sont calculés de telle façon à ce que, pour 1 équivalent molaire de 2,4-*bis*[p-diméthylamino)styryl]-quinoléine, 400 équivalents molaires de toxique soit rajoutés. Les scans sont réalisés à 5 min pour être comparés à un scan d'un témoin (à savoir, le puits comprenant uniquement la solution de 2,4-*bis*[p-diméthylamino)styryl]-quinoléine).

En phase liquide en solution dans le DMSO, la 2,4*-bis*[p-diméthylamino)styryl]-quinoléine change de couleur de façon radicale en 5 minutes, du jaune au noir, pour les cinq composés toxiques de guerre testés (Sarin, Tabun, Ypérite soufrée (HD), Ypérite azotée (HN3), Léwisite (L1)).

### EXEMPLE 4

Cet exemple illustre la mise en œuvre du composé préparé à l'exemple 1 pour la détection en phase supportée de composés pesticides organophosphorés.

Plus spécifiquement, deux prototypes de support ont été réalisés par « spotting » (plus spécifiquement, ici, par dépôt liquide de 13 gouttes distinctes du composé spécifique préalablement solubilisé à 10 mmol/L dans du DMSO à l'aide d'une pipette électronique multi-canaux suivi d'un séchage desdites gouttes), respectivement, un papier chromatographie (constitué de cellulose Grade 4 Chr, 460*570 mm et une épaisseur de 0,21 mm) et une lingette en fibres intissées (fibres de polyester et de cellulose Spec-Wipe 3 300*300 mm).

Les deux supports ainsi recouverts de 13 gouttes distinctes sont exposés à 12 pesticides (1 goutte de pesticide distinct pour une goutte de composé de l'exemple 1, la treizième goutte n'étant pas exposée pour servir de témoin), les 12 pesticides étant les suivants : Malathion, Phosalone, Trichlorfon, Ométhoate, Fenthion, Parathion, Chlorpyrifos, Paraoxon Méthyl, diméthoate, dichlorvos et diazinon.

Plus spécifiquement, chaque pesticide avant dépôt est préalablement solubilisé à 100 mmol/L dans du DMSO, lesdits pesticides étant ensuite déposés à raison de 1,2 µL *via* une pipette électronique multicanaux de sorte à exposer, au sein d'une goutte de composé de l'exemple 1, ledit composé à 10 équivalents de composé pesticide.

Les deux supports sont ensuite scannés après 5 minutes, 1 heure et 72 heures après exposition aux pesticides.

A l'issue des 72 heures, le virage colorimétrique s'est produit pour chacun des pesticides (virage du jaune brun au marron) pour les deux supports, avec un virage plus marqué pour la lingette en fibres intissées.

### EXEMPLE 5

Cet exemple illustre la mise en œuvre du composé préparé à l'exemple 1 pour la détection en phase supportée de composés toxiques de guerre.

Plus spécifiquement, deux prototypes de support sont réalisés par impression jet d'encre sur une imprimante de marque DIMATIX, un support en fibres intissées (fibres de polyester et fibres de cellulose) et un support en polyoléfine. Pour ce faire, la 2,4-*bis*[p-diméthylamino)styryl]-quinoléine est solubilisée à 100 mM dans le DMSO. L'impression est réalisée au moyen d'une imprimante jet d'encre Dimatix DMP 2800 series (FujiFilm) (Imprimante Dimatix (Fujifilm)). Les motifs d'impression sont des alvéoles de 8 mm de diamètre, prévoyant l'impression de gouttelettes espacées de 150 µm les unes des autres. Ce pas d'impression de 150 µm permet d'obtenir une densité de points imprimés, et donc de couleur, maximale. Dans notre cas, 1/3 des gouttelettes occupe 1/3 de la surface, les 2/3 restants de la surface n'étant pas imprimés.

Les deux supports ainsi recouverts d'alvéoles (11 au total) sont exposés à 10 composés toxiques de guerre (1 goutte de composé toxique de guerre distinct pour une alvéole distincte, la onzième alvéole n'étant pas exposée pour servir de témoin), les 10 composés toxiques de guerre étant les suivants : Sarin, Tabun, phosphonamidofluoridate de méthyl(1-(diéthylamino)éthylidène), phosphoramidofluoridate d'éthyl(1-(diéthylamino)éthylidène), (*bis*(diéthylamino)méthylène)phosphoramidofluoridate de méthyle, Ypérite soufrée (HD), Ypérite azotée (HN3), Léwisite (L1), diphénylchlorarsine (C1), diphénylcyanoarsine (C2).

Quel que soit le support, il est observé un virage colorimétrique des alvéoles après 5 minutes d'exposition pour chacun des composés toxiques de guerre.

### EXEMPLE 6

Cet exemple illustre la préparation de particules de silice imprégnées par le 2,4-*bis*[p-diméthylamino)styryl]quinoléine selon le protocole opératoire suivant :
1°) Pesée de la poudre dans un ballon ;
2°) Pesée de la quantité du composé quinoléine nécessaire (0,1% massique par rapport à la masse de poudre) dans un pilulier ;
3°) Ajout d'un solvant permettant la solubilisation du composé dans le pilulier et de sorte à avoir une concentration d'environ 30 mg/mL ;
4°) Versement de la solution de composé dans le ballon sur la poudre et rinçage du pilulier à plusieurs reprises et versement du solvant de rinçage dans le ballon ;
5°) selon l'aspect de la poudre dans le ballon, rajout d'autant de solvant que nécessaire pour mouiller et mettre en suspension la poudre ;
6°) Evaporation en trois étapes :
   *Mise en rotation dans le bain-marie (55°C) de l'évaporateur rotatif pendant 10 minutes pour homogénéiser la suspension dans le ballon et monter en température ;
   *Descente en pression lente jusqu'à 400 mbar pour distiller lentement le solvant ;
   *Lorsque la poudre commence à se regrouper pour former un anneau qui s'attache à la paroi du ballon, la pression est descendue plus rapidement jusqu'au séchage complet.

La poudre imprégnée ainsi obtenue est incorporée dans une solution obtenue selon le mode opératoire suivant :
1°) Préparation d'une solution de copolymère bloc polystyrène-b-poly(éthylène-ran-butylène)-b-polystyrène (dit PSPEPB) à 30% massique dans le cyclohexane ;
2°) Pesée du décane dans un flacon en verre ;
3°) Ajout de la masse nécessaire de solution de PSPEPB ;
4°) Ajout de la masse nécessaire de poudres imprégnées dans le flacon ;
5°) Mélange par agitation vortex et ultrasons si nécessaire.

Le tableau ci-dessous illustre, plus précisément, les proportions massiques de ces ingrédients en fonction de la nature de la poudre utilisée.

| Poudre | Composition |
|---|---|
| Silice | 30% de poudre imprégnée, 9,6% de PSPEPB, 22,4% de cyclohexane, 38% décane |

La composition est déposée ensuite sur un substrat en papier chromatographie par enduction à la racle et plus spécifiquement, au moyen d'un outil de raclage semi-automatique Elcometer 4340 *via* les opérations suivantes :
- fixation de la vitesse de la racle au minimum (position 1 sur les 11 positions envisageables) ;
- fixation du substrat sur la table métallique de l'outil ;
- dépôt manuel d'un bourrelet de la composition de particules devant la racle à la pipette Pasteur en polypropylène ;
- entraînement de la racle à la vitesse programmée par le portique mobile ;
- détachement du substrat puis séchage sous sorbonne.

Le substrat ainsi obtenu est ensuite soumis à 12 composés toxiques déposés chacun, à raison de 1,6 µL *via* une pipette électronique multicanaux, dans des alvéoles individuelles prévues sur le support. Le support est ensuite scanné après 5 minutes puis après 1 heure d'exposition aux composés toxiques.

Les 12 composés toxiques sont des composés organophosphorés de la série G (Sarin, Soman et Tabun), un composé organosphosphoré de la série V (le VX, CAS n°50782-69-9), des vésicants (l'ypérite soufrée, l'ypérite azotée, la léwisite), des composés arséniés (la diphénylchlorarsine dit « C1 » et la diphénylcyanoarsine dit « C2 ») et les composés organophosphorés suivants : le phosphonamidofluoridate de méthyl(1-(diéthylamino)éthylidène), le phosphoramidofluoridate de méthyl(1-(diéthylamino)éthylidène), le phosphoramidofluoridate d'éthyl(1-(diéthylamino)éthylidène).

Le substrat susmentionné permet la détection des 12 composés toxiques de la manière suivante :
- par l'apparition d'une couleur plus foncée (brun bordeau) au contact des composés toxiques (Tabun, Ypérite soufrée (HD), Ypérite azotée (HN3), diphénylcyanoarsine (C2)) ;
- par l'éclaircissement vers le jaune au contact des composés toxiques (VX, phosphoramidofluoridate de méthyl(1-(diéthylamino)éthylidène), phosphonamidofluoridate de méthyl(1-(diéthylamino)éthylidène), phosphoramidofluoridate d'éthyl(1-(diéthylamino)éthylidène), Léwisite (L1), diphénylchlorarsine (C1)) ; ou
- par un faible changement de la couleur initiale au contact des composés toxiques (Sarin, Soman).

Des essais ont également été réalisés avec des particules de polyamide-6, de polyamide-12, de polyéthylène, de silice fonctionnalisée par des groupes amines avec également des résultats probants pour les 12 composés toxiques susmentionnés.

## Revendications

1. Utilisation d'un composé comprenant un groupe aromatique comprenant au moins un cycle aromatique azoté, lequel cycle étant porteur de deux substituants identiques qui répondent à la formule (I) suivante : 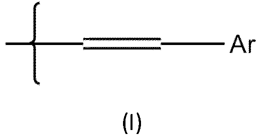
dans laquelle Ar désigne un groupe aromatique éventuellement substitué et l'accolade désigne l'endroit par lequel le substituant est lié au cycle aromatique azoté,
ledit composé étant utilisé comme indicateur de détection pour la détection d'au moins un composé chimique choisi parmi les composés organophosphorés, les composés sulfures, les composés amines et les composés arséniés.

2. Utilisation selon la revendication 1, dans laquelle le composé comprend, comme groupe aromatique comprenant au moins un cycle aromatique azoté, un groupe aromatique comprenant un seul cycle aromatique azoté.

3. Utilisation selon la revendication 1 ou 2, dans laquelle le composé comprend, comme groupe aromatique comprenant au moins un cycle aromatique azoté, un groupe aromatique comprenant, comme cycle aromatique azoté, un cycle pyridine.

4. Utilisation selon l'une quelconque des revendications précédentes, dans laquelle le groupe aromatique est un groupe pyridine ou un groupe quinoléine.

5. Utilisation selon l'une quelconque des revendications 1 à 4, dans laquelle le groupe aromatique Ar est un groupe aromatique monocyclique carboné, de préférence, un groupe phényle.

6. Utilisation selon l'une quelconque des revendications 1 à 4, dans laquelle le groupe aromatique Ar est un groupe hétéroaromatique monocyclique.

7. Utilisation selon la revendication 6, dans laquelle le groupe aromatique Ar est un groupe hétéroaromatique azoté monocyclique, de préférence, un groupe pyridine.

8. Utilisation selon l'une quelconque des revendications 1 à 7, dans laquelle le groupe aromatique Ar est substitué par un ou plusieurs substituants.

9. Utilisation selon la revendication 8, dans laquelle le ou les substituants sont choisis parmi un substituant amine, un substituant alcoxy, un substituant hydroxyle -OH, un substituant nitro -NO₂, un substituant halogène, un substituant alkyle halogéné, un substituant ester, un substituant acide carboxylique -COOH, un substituant cyano -CN ou un substituant -S-alkyle.

10. Utilisation selon la revendication 1, dans laquelle le composé est un composé comprenant, comme groupe aromatique comprenant au moins un cycle aromatique azoté, un groupe quinoléine, le cycle aromatique azoté du groupe quinoléine étant porteur de deux substituants identiques comprenant chacun une double liaison conjuguée avec ledit cycle et comprenant chacun un groupe aromatique conjugué avec ladite double liaison.

11. Utilisation selon l'une quelconque des revendications 1 à 4, dans laquelle le composé répond à la formule (II) suivante : 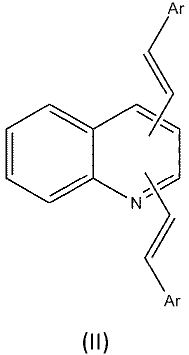 dans laquelle Ar désigne un groupe aromatique éventuellement substitué par un groupe choisi parmi un substituant amine, un substituant alcoxy, un substituant hydroxyle -OH, un substituant nitro -NO₂, un substituant halogène, un substituant alkyle halogéné, un substituant ester, un substituant acide carboxylique -COOH, un substituant cyano -CN ou un substituant -S-alkyle.

12. Utilisation selon la revendication 11, dans laquelle le composé répond à la formule (III) suivante : 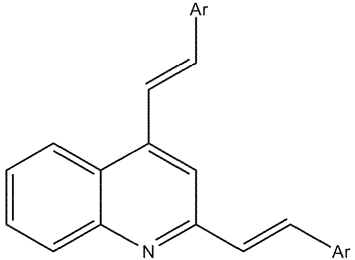 avec Ar étant tel que défini à la revendication 11.

13. Utilisation selon la revendication 11 ou 12, dans laquelle le composé répond à la formule (IV) suivante :

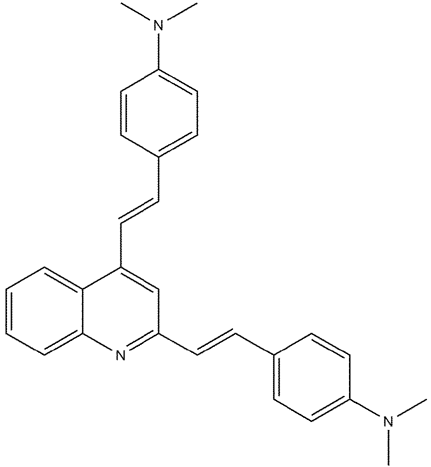

14. Utilisation selon l'une quelconque des revendications précédentes, dans laquelle le composé utilisé comme indicateur de détection est utilisé en phase liquide ou en phase supportée.

15. Procédé de détection de la présence ou de l'absence d'au moins un composé chimique choisi parmi les composés organophosphorés, les composés sulfures, les composés amines et les composés arséniés comprenant les étapes suivantes :
- une étape de mise en contact du ou des milieux, dont on veut détecter la présence ou l'absence du ou desdits composés chimiques avec un indicateur de détection qui est un composé tel que défini selon l'une quelconque des revendications 1 à 14 ;
- une étape de déduction, en fonction du ou des éventuels signaux de transformation de l'indicateur de détection, de la présence ou de l'absence du ou desdits composés.

## Patentansprüche

1. Verwendung einer Verbindung, eine aromatische Gruppe umfassend, die mindestens einen stickstoffhaltigen aromatischen Ring umfasst, wobei der Ring Träger von zwei identischen Substituenten ist, die der folgenden Formel (I) entsprechen: 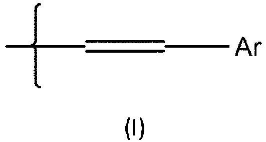
wobei Ar eine eventuell substituierte aromatische Gruppe bezeichnet und die geschweifte Klammer die Stelle bezeichnet, durch die der Substituent an den stickstoffhaltigen aromatischen Ring gebunden ist,
wobei die Verbindung als Detektionsindikator zur Detektion mindestens einer chemischen Verbindung verwendet wird, die aus organischen Phosphorverbindungen, Sulfidverbindungen, Aminverbindungen und arsenhaltigen Verbindungen ausgewählt ist.

2. Verwendung nach Anspruch 1, wobei die Verbindung als aromatische Gruppe, die mindestens einen stickstoffhaltigen aromatischen Ring umfasst, eine aromatische Gruppe umfasst, die einen einzigen stickstoffhaltigen aromatischen Ring umfasst.

3. Verwendung nach Anspruch 1 oder 2, wobei die Verbindung als aromatische Gruppe, die mindestens einen stickstoffhaltigen aromatischen Ring umfasst, eine aromatische Gruppe umfasst, die als stickstoffhaltigen aromatischen Ring einen Pyridinring umfasst.

4. Verwendung nach einem der vorstehenden Ansprüche, wobei die aromatische Gruppe eine Pyridingruppe oder eine Chinolingruppe ist.

5. Verwendung nach einem der Ansprüche 1 bis 4, wobei die aromatische Gruppe Ar eine kohlenstoffhaltige monozyklische aromatische Gruppe, vorzugsweise eine Phenylgruppe ist.

6. Verwendung nach einem der Ansprüche 1 bis 4, wobei die aromatische Gruppe Ar eine monozyklische heteroaromatische Gruppe ist.

7. Verwendung nach Anspruch 6, wobei die aromatische Gruppe Ar eine monozyklische heteroaromatische stickstoffhaltige Gruppe, vorzugsweise eine Pyridingruppe ist.

8. Verwendung nach einem der Ansprüche 1 bis 7, wobei die aromatische Gruppe Ar durch einen oder mehrere Substituenten substituiert ist.

9. Verwendung nach Anspruch 8, wobei der oder die Substituenten aus einem Aminsubstituenten, einem Alkoxysubstituenten, einem Hydroxylsubstituenten -OH, einem Nitro-Substituenten -NO₂, einem Halogensubstituenten, einem Halogenalkylsubstituenten, einem Estersubstituenten, einem Carboxylsäuresubstituenten -COOH, einem Cyansubstituenten -CN oder einem Alkylsubstituenten -S ausgewählt sind.

10. Verwendung nach Anspruch 1, wobei die Verbindung eine Verbindung ist, die als aromatische Gruppe, die mindestens einen stickstoffhaltigen aromatischen Ring, eine Chinolingruppe umfasst, wobei der stickstoffhaltige aromatische Ring der Chinolingruppe Träger von zwei identischen Substituenten ist, die jeweils eine mit dem Ring konjugierte Doppelbindung umfassen und jeweils eine mit der Doppelbindung konjugierte aromatische Gruppe umfassen.

11. Verwendung nach einem der Ansprüche 1 bis 4, wobei die Verbindung der folgenden Formel (II) entspricht: 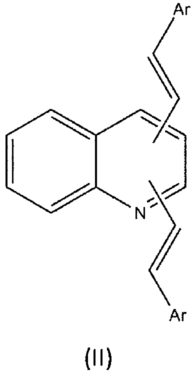 wobei Ar eine eventuell durch eine Gruppe substituierte aromatische Gruppe bezeichnet, die aus einem Aminsubstituenten, einem Alkoxysubstituenten, einem Hydroxylsubstituenten -OH, einem Nitro-Substituenten -NO₂, einem Halogensubstituenten, einem Halogenalkylsubstituenten, einem Estersubstituenten, einem Carboxylsäuresubstituenten -COOH, einem Cyansubstituenten -CN oder einem Alkylsubstituenten -S ausgewählt ist.

12. Verwendung nach Anspruch 11, wobei die Verbindung der folgenden Formel (III) entspricht: 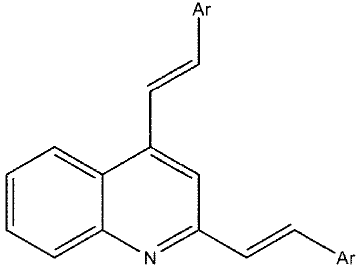 wobei Ar gemäß Anspruch 11 definiert ist.

13. Verwendung nach Anspruch 11 oder 12, wobei die Verbindung der folgenden Formel (IV) entspricht:

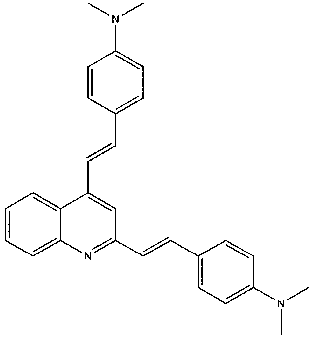

14. Verwendung nach einem der vorstehenden Ansprüche, wobei die als Detektionsindikator verwendete Verbindung in flüssiger Phase oder in Trägerphase verwendet wird.

15. Verfahren zur Detektion des Vorhandenseins oder Nicht-Vorhandenseins mindestens einer chemischen Verbindung, die aus organischen Phosphorverbindungen, Sulfidverbindungen, Aminverbindungen und arsenhaltigen Verbindungen ausgewählt ist, die folgenden Schritte umfassend:
- einen Schritt zum In-Kontakt-Bringen des oder der Medien, bei denen man das Vorhandensein oder Nicht-Vorhandensein der einen oder der mehreren chemischen Verbindungen mit einem Detektionsindikator detektieren will, der eine Verbindung wie gemäß einem der Ansprüche 1 bis 14 definiert ist;
- einen Schritt zum Detektieren in Abhängigkeit von dem oder den eventuellen Transformationssignalen des Detektionsindikators, des Vorhandenseins oder Nicht-Vorhandenseins der Verbindung(en).

## Claims

1. Use of a compound comprising an aromatic group comprising at least one nitrogenous aromatic cycle, which cycle carries two identical substituents which correspond to the following formula (I): 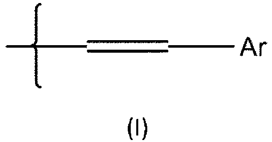
wherein Ar denotes an optionally substituted aromatic group and the bracket indicates the position at which the substituent is bonded to the nitrogenous aromatic cycle,
said compound being used as a detection indicator for the detection of at least one chemical compound selected from organophosphate compounds, sulphide compounds, amine compounds and arsenic-containing compounds.

2. Use according to claim 1, wherein the compound comprises, as an aromatic group comprising at least one nitrogenous aromatic cycle, an aromatic group comprising a single nitrogenous aromatic cycle.

3. Use according to claim 1 or 2, wherein the compound comprises, as an aromatic group comprising at least one nitrogenous aromatic cycle, an aromatic group comprising, as a nitrogenous aromatic cycle, a pyridine cycle.

4. Use according to any one of the preceding claims, wherein the aromatic group is a pyridine group or a quinoline group.

5. Use according to any one of claims 1 to 4, wherein the aromatic group Ar is a carbonated monocyclic aromatic group, preferably a phenyl group.

6. Use according to any one of claims 1 to 4, wherein the aromatic group Ar is a monocyclic heteroaromatic group.

7. Use according to claim 6, wherein the aromatic group Ar is a monocyclic nitrogenous heteroaromatic group, preferably a pyridine group.

8. Use according to any one of claims 1 to 7, wherein the aromatic group Ar is substituted with one or more substituents.

9. Use according to claim 8, wherein the substituent(s) is/are selected from an amine substituent, an alkoxy substituent, a hydroxyl substituent -OH , a nitro substituent -NO₂, a halogen substituent, a halogenated alkyl substituent, an ester substituent, a carboxylic acid substituent -COOH, a cyano substituent -CN, or an - S-alkyl substituent.

10. Use according to claim 1, wherein the compound is a compound comprising, as an aromatic group comprising at least one nitrogenous aromatic cycle, a quinoline group, the nitrogenous aromatic cycle of the quinoline group carrying two identical substituents, each comprising a double bond conjugated with said cycle and each comprising an aromatic group conjugated with said double bond.

11. Use according to any one of claims 1 to 4, wherein the compound complies with the following formula (II): 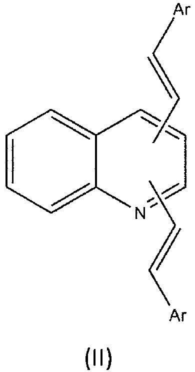 wherein Ar denotes an aromatic group optionally substituted by a group chosen from an amine substituent, an alkoxy substituent, a hydroxyl substituent - OH, a nitro substituent -NO₂ , a halogen substituent, a halogenated alkyl substituent, an ester substituent, a carboxylic acid substituent -COOH, a cyano substituent -CN or an -S-alkyl substituent.

12. Use according to claim 11, wherein the compound complies with the following formula (III): 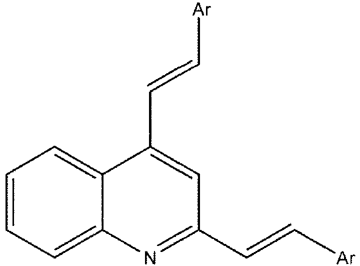 with Ar being as defined in claim 11.

13. Use according to claim 11 or 12, wherein the compound complies with the following formula (IV):

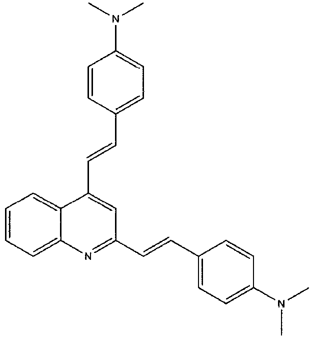

14. Use according to any one of the preceding claims, wherein the compound used as a detection indicator is used in the liquid phase or supported phase.

15. Method for detecting the presence or absence of at least one chemical compound selected from organophosphate compounds, sulphide compounds, amine compounds and arsenic-containing compounds comprising the following steps:
- a step of placing the medium/media in contact, it being desirable to detect the presence or absence of said chemical compound(s) therein with a detection indicator which is a compound as defined according to any one of claims 1 to 14;
- a step of deducing, according to the possible transformation signal(s) of the detection indicator, the presence or absence of said compound(s).
